# EUROPEAN PATENT APPLICATION

(11) **EP 0 842 638 A2**
(43) Date of publication of application: **20.05.1998**
(21) Application number: 97308964.2
(22) Date of filing: 07.11.1997
(51) Int. Cl.: A61B 8/06

(54) **Ultrasonic diagnostic imaging system with real time volume flow calculation**

(30) Priority: 08.11.1996 US 29542 P
(71) Applicant: ATL Ultrasound, Inc., Bothell, Washington 98041 (US)
(72) Inventor: Routh, Helen F., Seattle, Wa 98105 (US); Robinson, Marshall T., Snohomish, Washington 98290 (US)
(74) Representative: Mercer, Christopher Paul

(57) **Abstract**

An ultrasonic diagnostic imaging system is provided for making volume flow measurements in the heart and blood vessels of the body. The user places a cursor over the region of an imaged vessel where a flow measurement is to be made. The cursor is controlled to indicate the direction of the blood flow and the diameter of the vessel at the measurement point. The cursor also defines a sample volume at which Doppler velocity measurements are made. The velocity measurements and vessel diameter are used to compute the vessel cross sectional area and volume flow of blood at the measurement point. In a preferred embodiment the cursor continually automatically tracks the vessel walls even in the presence of vessel pulsatility and ultrasonic probe movement.

## Description

This invention relates to ultrasonic diagnostic systems which measure blood flow characteristics of the body and, in particular, to the measurement of blood vessels and their blood flow characteristics.

Ultrasonic Doppler techniques have long been used to noninvasively measure the flow of blood in the heart and blood vessels of the body. In the practice of the Doppler technique, waves of returning ultrasonic signals are compared to a phase reference to determine the phase shift of the returning waves. As the transmitted ultrasonic wave impinges upon flowing material such as blood cells, the movement of the flowing material will impart a Doppler shift to the returning echo signal. This frequency shift, which is commonly measured in kilohertz, translates into the rate of movement or velocity of the blood flow. Doppler velocity information is conventionally displayed as a continuous spectrum of lines of varying amplitudes in a moving or scrolled display on a video monitor, as shown in U.S. patents 5,634,465 and 5,287,753. Each line represents an instantaneous measurement of blood flow velocities. As the flow of blood in a vessel or the heart is continuously monitored and interrogated by Doppler ultrasound, the spectrum of systolic and diastolic velocities is continuously displayed and passed before the clinician.

It has long been desirable to extend the Doppler technique, which measures the speed of blood flow, to applications where the technique is used in the computation of the volume of blood flowing through a vessel, or cardiac output. One technique which has been developed for computing the volume flow of blood combines a measurement of the radius or diameter of a blood vessel with the mean velocity of blood flow. The blood vessel measurement is used to estimate the cross-sectional area of the vessel, and the product of this area and the mean velocity of blood flow yields an estimate of the volume of blood passing through the vessel in a unit of time.

Measuring the radius or diameter of a vessel requires care, as the accuracy of the measurement determines the accuracy of the computed vessel cross-sectional area and the subsequent volume flow calculation. In particular, it is necessary to make the measurement through the center of the vessel, so that the measurement is of the true diameter or radius and not of a chord across the vessel. Measuring a chord rather than the radius or diameter will underestimate the area and hence the volume of blood flow. In addition, the Doppler measurement should be taken across the entire vessel at the point of the diameter or radius measurement, and should be calibrated in accordance with the direction of the blood flow in relation to the ultrasound transducer, as Doppler measurements are known to be angle dependent. Furthermore, the volume flow measure should consider the velocity of blood flow throughout the full cardiac cycle for optimal accuracy.

In accordance with the principles of the present invention, apparatus and methods are presented for measuring the size of a blood vessel and computing the volume of blood flowing through the vessel. An ultrasonic imaging system employs a user adjustable cursor which is used to measure a blood vessel. The cursor includes one segment which can be aligned with the direction of blood flow through the vessel, and another segment aligned with the first which is used to measure the diameter or radius of the vessel. The cursor defines a sample volume region in which Doppler measurements are taken of the full cross sectional area of the vessel. In one embodiment the cursor is used in combination with Doppler measurements to compute the volume of blood flowing through the vessel. In a preferred embodiment the volume flow computation is based upon the blood flow velocities over a full cardiac cycle, and is recomputed each cardiac cycle. In another preferred embodiment the cursor automatically tracks variations in the size of the vessel with blood flow pulsatility. This changing cursor measurement is used to make instantaneous volume flow measures, which are aggregated to compute volume flow per heart cycle or average volume flow over a known time interval.

In the drawings:
FIGURE 1 illustrates in block diagram form an ultrasonic diagnostic imaging system constructed in accordance with the principles of the present invention;
FIGURE 2 illustrates a two dimensional and spectral Doppler ultrasound display illustrating volume flow measurements made in accordance with the principles of the present invention;
FIGURE 3 illustrates the use of a measurement cursor of the present invention in conjunction with an M-mode display; and
FIGURE 4 is an enlarged view of a measurement cursor of the present invention.

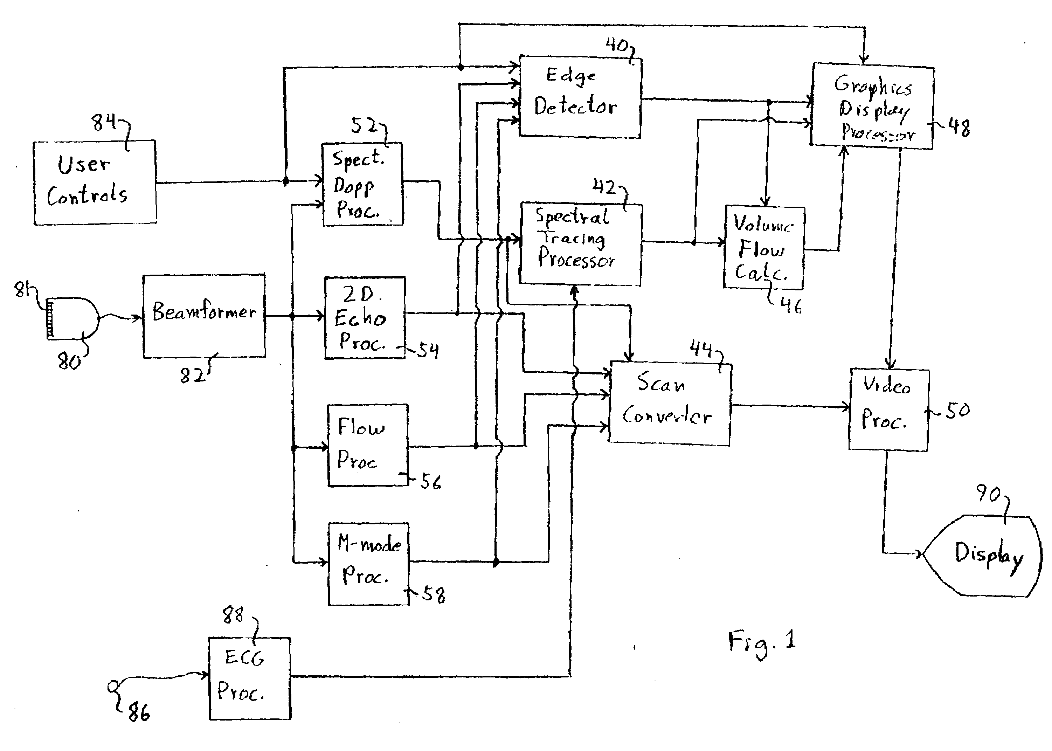

Referring to FIGURE 1, an ultrasonic diagnostic imaging system is shown which makes calculations of the volume flow of blood through blood vessels or the cardiac output of the heart in real time. Ultrasonic waves are transmitted into the body of a patient by the transducer array 81 of an ultrasonic probe 80, which receives returning echoes from the impingement of the waves on tissue and blood cells within the body. The echoes received by the elements of the probe transducer are combined into a focused received beam by a beamformer 82. The received echoes may be Doppler processed to form a spectral Doppler display by a spectral Doppler processor 52, or amplitude detected and used to form a two dimensional image of the structure within the body by a 2D echo processor 54, or Doppler processed by a flow processor 56 to produce a colorflow image of flow velocities in conjunction with a two dimensional image, or a single spatial beamline can be repetitively displayed in time by an M-mode processor 58. The processed spectral Doppler, 2D, colorflow, and M-mode signals are all coupled to a scan converter 44, which converts the signals into the desired image display format. The image display signals are applied to a video processor 50, which produces signals for display of the ultrasound image on a display 90.

The spectral Doppler signals are also coupled to a spectral tracing processor 42, which computes the mean and peak velocities of the spectral display in real time as described in U.S. Pat. 5,287,753 or U.S. Pat 5,634,465. The spectral tracing processor 42 also receives a heartbeat waveform from ECG processor 88, which receives the patient's heartbeat by means of an ECG lead 86. The spectral display lines with mean and peak velocity real time tracings are coupled to a graphics display processor 48 which arranges graphical and alphanumeric display information into a desired display format. The spectral display graphics are coupled to the video processor 50 for display on the display 90.

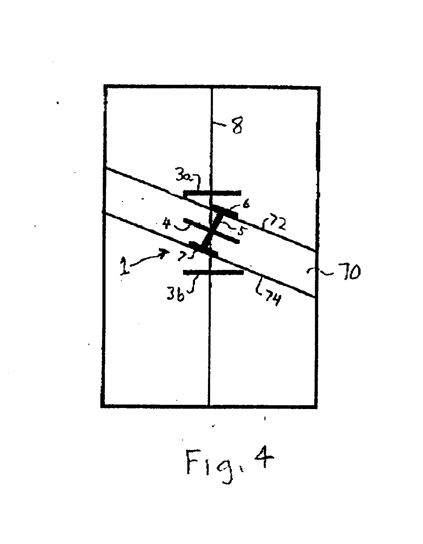

The system of FIGURE 1 computes the volume flow of blood through a blood vessel or orifice by making a 2D, colorflow, or M-mode measurement of the cross sectional area of a vessel or orifice, and multiplying this area by the average flow velocity through the vessel or orifice. It is important that the velocity measurement be made from the same site as the area measurement, which is often difficult to do as the probe or patient move and the 2D view changes. When the user has obtained a two dimensional image of a blood vessel 70 on the screen of display 90 as shown in FIGURE 4, a cursor 1 is positioned on the vessel where the volume flow is to be computed. In FIGURE 4 the vessel 70 is shown in cross section and has two walls 72 and 74 which are visible in the image. The cursor 1 is contained within sample volume lines 3a and 3b which define a sample volume in which a volume flow measurement is to be made.

The cursor 1 contains four components. A flow direction line 4 is positioned by the user in line with the direction of blood flow through the vessel 70. Placement of the flow direction line 4 informs the ultrasound system of the relationship between the ultrasound beam direction 8 and the direction of flow through the vessel, enabling the system to correct for a non-zero angle of incidence. Furthermore, since the direction of flow is assumed to be parallel to the walls 72,74 of the vessel, alignment of the line 4 with the direction of flow assures that a second line 5, orthogonal to the flow direction line 4, is orthogonal to the walls of the vessel. This is important, as the line 5 is a line which is intended to delineate the diameter of the vessel 70. Since the diameter of the vessel must be normal to the vessel walls, the orthogonal relationship between the diameter line 5 and the flow direction line 4 assures that the line 5 depicts the vessel diameter, and not some skewed chord line across the vessel. Correspondingly, when the diameter line 5 is normal to the vessel walls 72,74, the orthogonal flow direction line is properly aligned with the direction of flow.

At the ends of the diameter line 5 are wall lines 6 and 7, which the user places on the walls 72, 74 of the vessel in the display. The user controls permit the user to lengthen or shorten the diameter line 5 and relocate the diameter line until the wall lines 6 and 7 are aligned with the vessel walls 72, 74. When so placed with the image plane passing through the center of the vessel, the diameter line 5 accurately represents the diameter of the blood vessel.

The cursor 1 which is used to measure the vessel area is contained within the sample volume 3a,3b used for flow measurement, thereby assuring that the area and velocity measurements are taken at the same point in the blood vessel. Since the cursor 1 is entirely within the sample volume delineated by 3a,3b, Doppler samples taken across the sample volume will necessarily be taken from across the entire blood vessel, assuring an accurate measurement of blood flow velocity through the vessel. As the user adjusts the length of the diameter line 5 to match the diameter of a vessel, the sample volume delineations 3a,3b automatically adjusts so that the entire diameter of the vessel always fits within the sample volume.

As mentioned above, it is important for the diameter line 5 of the cursor to pass through the center of the vessel 70 and not through a shorter chord of the vessel circumference, if the line 5 is to accurately represent the vessel diameter. This may be achieved by aligning the scan plane of the image of FIGURE 4 so that it slices through the cross sectional center of the vessel at the location of cursor 1. This may be done by visually observing the vessel diameter as the scan plane is swept across the vessel through movement of the probe 80. When the vessel appears to have the largest cross sectional diameter, the user can assume that the scan plane is slicing through the vessel diameter.

User adjustment of the cursor 1 on the ultrasound system display can be performed using the conventional trackball, select key and other controls of the system user controls 84. The trackball is first manipulated to place the cursor 1 at the center of the vessel to be measured. A paddle switch of the user controls is actuated to incrementally rotate the cursor until the direction line 4 is aligned with the direction of blood flow through the vessel.. A "distance" key is depressed to commence a diameter measurement. The trackball is manipulated to shorten or lengthen the diameter line 5 until the wall lines 6,7 are aligned with the vessel walls. The select key is depressed to retain each wall line in its selected position. The sample volume lines 3,3b automatically move with changes in the wall lines 6,7 so as to constantly encompass the cursor lines 4,5. The ultrasound system can now compute volume flow through the vessel at the location of the cursor.

The parameters associated with the cursor 1 including the diameter line 5 are coupled to the graphics display processor 48, and to a volume flow calculator 46 by means of an edge detector 40. The graphics display processor 48 uses the cursor location and dimension parameters to accurately display the cursor 1 and sample volume on the ultrasound image where the vessel is being measured. The volume flow calculator receives mean bloodflow velocity values from the spectral tracing processor 42 and the diameter of the vessel in the sample volume from the edge detector. The vessel diameter is used to compute the area of the vessel, and the area is multiplied by the bloodflow velocity to produce a volume flow measurement in real time. This measurement is also coupled to the graphics display processor 48 for display in real time.

The edge detector can simply pass the diameter measure to the volume flow calculator, but preferably the edge detector is operated to cause the wall lines 6 and 7 to maintain their alignment with the vessel walls 72 and 74. The edge detector is initially trained to recognize the vessel walls by the user's placement of the wall lines on the image, giving the edge detector the starting reference locations from which to track any movement of the vessel walls in the image, as well as an indication of the image characteristics which represent the vessel walls. Movement of the vessel walls in the image may result from pulsatory contraction and expansion of the vessel with the pumping of blood, or from movement of the transducer probe 80 in the hand of the user, for example. The edge detector tracks any such movement and maintains the placement of the wall lines 6 and 7 on the vessel walls. The edge detector does this through detection of the vessel walls by any of a number of techniques. The edge detector may detect the transition between bloodflow and the vessel wall through analysis of Doppler or colorflow signal differences in the vicinity of the wall lines. Or, the edge detector may detect the transition between echoes from blood cells to echoes from tissue in the vicinity of the wall lines. A number of edge detecting algorithms can automatically find the vessel walls given a starting location in the center of the vessel. Or, the edge detector may track the peak flow velocity or vessel diameter and correspondingly adjust the lateral positioning of the cursor 1 or elevation steering of the image plane to keep the cursor centered on the vessel. The edge detector 40 thus continues to track the vessel walls, maintaining the wall lines 6 and 7 in alignment with the walls, and lengthens and shortens the diameter line 5 in correspondence with any changes. These changes to the cursor and the diameter line are coupled to the graphics display processor for real time display of these cursor changes, and are also coupled to the volume flow calculator 46 to maintain the accuracy of the real time volume flow calculation.

The ability to control the ultrasound beam in the elevation plane allows for even more precise control of volume flow measurements. Using the user-set diameter line 5 or corresponding sample volume lines 3a,3b, and assuming that the vessel is circular, the size of the sample volume in the elevation dimension is controlled by elevation focusing. The elevation slice thickness is controlled to encompass the vessel diameter in the elevation direction (in addition to setting the cursor in the image plane dimension). Thus, with the sample volume size closely aligned with the vessel diameter in both the image plane and the elevation dimensions, even more accurate volume flow measurements can be obtained.

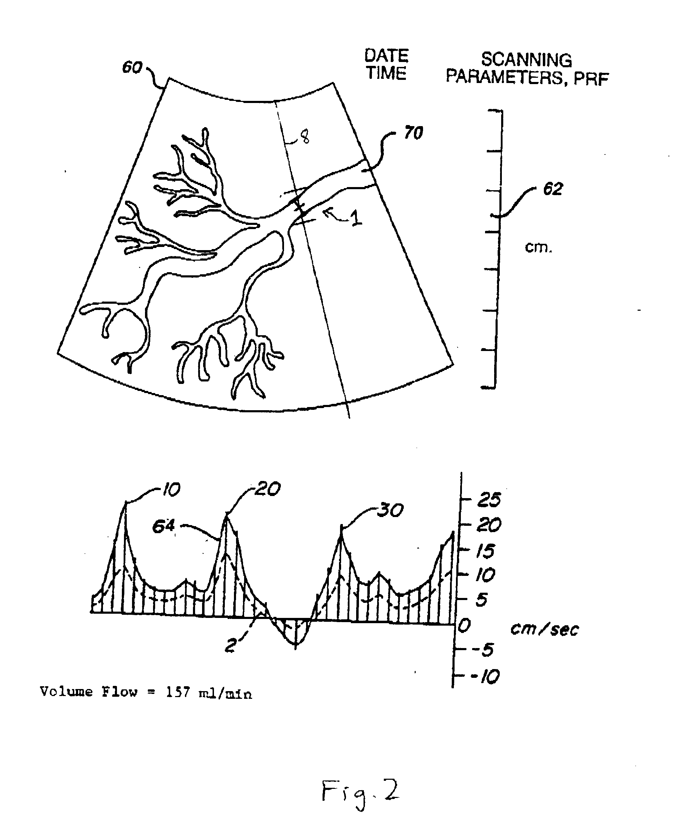

FIGURE 2 depicts a typical duplex screen display on the display 90. At the top of the display is a 2D ultrasound image 60 including a displayed blood vessel 70. The cursor 1 is located on a beam line 8 and the user controls are manipulated as described above to locate the cursor 1 on the blood vessel 70. The diameter of the vessel 70 is contained within the sample volume lines of the cursor, the flow direction line is aligned with the direction of blood flow through the vessel, and the diameter line is extended or shortened until the wall lines are aligned with the opposite walls of the vessel in the image. With the cursor 1 positioned in this manner, a spectral display is produced to scroll across the lower half of the display, on which are displayed a peak velocity line 64 and a dashed mean velocity line 2. The volume flow calculator then uses the dimension of the diameter line of the cursor on the ultrasound image and the mean flow velocity of the spectral display to compute a volume flow rate through the vessel in the sample volume, as shown at the bottom of the display. In this example the diameter line has produced a blood vessel diameter of 0.8 cm, the flow velocity is 5.2 cm/sec, and a volume flow of 157 ml/min is computed. The flow velocity is calculated by averaging the mean velocity measurements 2 over one cardiac cycle. In the illustrated example this would be the mean velocity values on each spectral line between peaks 10 and 20 or between peaks 20 and 30 of consecutive cardiac cycles. The mean velocities on each spectral line over the cardiac cycle are summed, then divided by the number of spectral lines to arrive at an average velocity over the heart cycle. The average velocity is then multiplied by the cross sectional area of the vessel to compute the volume flow through the vessel. The volume flow is recomputed each cardiac cycle, and the displayed value (*e.g.*, 157 ml/min) updated each heart cycle, or at some other interval chosen by the user. Alternatively, or additionally, a plot of the volume flow over time can be produced on the system display. The portion of the spectral display which illustrates the cardiac cycle used to compute the currently displayed volume flow value, *e.g.*, the spectral lines between peaks 10 and 20 or between peaks 20 and 30, may be highlighted in color or brightness or delineated by cursors on the image display, so that the operator will see which heart cycle was used to compute the measurement.

In a preferred embodiment, the user can first set the diameter line 5 on the vessel diameter. The system will then automatically set the sample volume size to be slightly larger than and encompass the diameter line.

In a preferred embodiment the 2D display 60 and the spectral display are both displayed in real time, with 2D and Doppler scanlines acquired in a time interleaved manner. This enables the user to constantly monitor the positioning of the cursor 1 over the vessel 70 while the spectral display scrolls across the screen and new volume flow measurements are updated with each heartbeat. The system may also be operated in a mode in which only one of the displays is live while the other is frozen on the screen. In use of such a display, the 2D image 60 is first operated in real time while the user places the cursor over the vessel 70. Once the cursor is properly located the user freezes the 2D image 60 and switches the spectral display to real time operation. The user can occasionally switch the 2D image back to real time operation from time to time to visually see whether the diameter line 5 has moved out of alignment with the vessel walls, a condition which is indicated when the spectral display amplitude drifts or changes, for instance. If the user sees that the diameter line has drifted from alignment with the vessel or the vessel has gotten smaller, the user will know that the probe or diameter line must be relocated to realign the diameter line with the diameter of the vessel. When the edge detector is employed to automatically and continuously track the alignment, the system can be controlled to audibly or visually notify the user when alignment is no longer being maintained. The user can also be notified in a tracking mode if the vessel and diameter line both shrink to a small size, an indication that the scan plane is no longer passing through the center of the vessel where the measurement should be made.

Since ultrasound studies are conventionally recorded on videotape and/or in Cineloop® memory, a recorded image sequence can be replayed and examined frame by frame to see that the cursor 1 is always in alignment with the vessel where a measurement is being made. If a frame or sequence of frames is found where the cursor is out of alignment with the vessel, the physician may consider disregarding the measurements of that sequence and acquiring a new image sequence for measurement data.

The preferred technique for computing volume flow with the embodiment in which vessel alignment is automatically tracked and maintained is to compute an instantaneous volume flow measurement with every new diameter or velocity data value during the heart cycle, then to average these instantaneous volume flow values over the heart cycle to arrive at a mean volume flow value for the heart cycle.

In addition to the volume flow calculation, the system of FIGURE 1 also can provide a cardiac output measure equal to the orifice area of a valve multiplied by the time averaged peak velocity, as the spectral tracing processor 42 provides both mean and time averaged peak flow velocities. For cardiac output measurements it is preferred to locate a small sample volume in the center of the flow and use the Doppler measurements at this location to determine the peak velocity with the spectral tracing processor. Cardiac output can be calibrated in heart cycles of flow by dividing cardiac output by the heart rate, yielding a measure called stroke volume. Stroke volume is a measure of the volume of blood pumped through a vessel or orifice in one heart cycle, generally shown in units of ml per heart cycle.

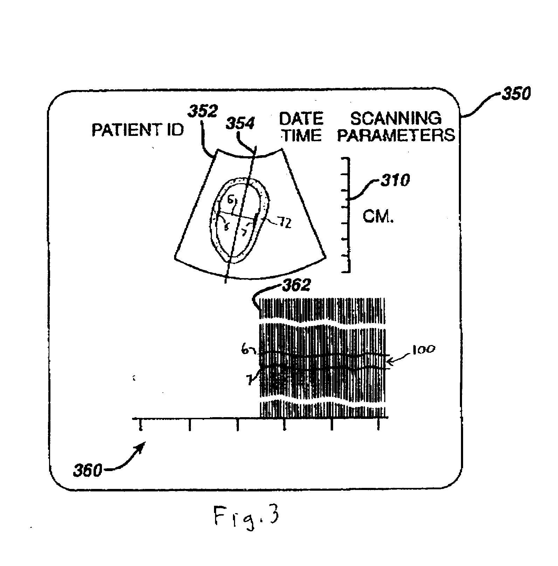

The 2D and M-mode display combination of FIGURE 3 can be employed to measure the volume of a chamber of the heart. Instead of imaging a blood vessel 70, the probe is directed to display an image plane of a heart chamber 72 in the 2D image 352. The M-line 354 is directed down the length of the chamber to automatically track or display the chamber measurement 100 in the M-mode display. The diameter line 5 of the cursor 1 is then set to measure the orthogonal dimension across the width of the chamber in the 2D image. These chamber length and width measures can then be combined in known algorithms, such as those fitting the measures as the major and minor axes of an assumed generally elliptical chamber shape, to compute an estimate of heart chamber volume.

## Claims

1. In an ultrasonic diagnostic imaging system which produces an ultrasonic display of blood vessels or organs, apparatus for measuring a dimension of a displayed blood vessel or organ comprising:
a display generator producing on said display a cursor having a first component for aligning with the direction of flow in a vessel or organ, and a second component, orthogonal to the first component, for aligning across the displayed vessel or organ orthogonal to said direction of flow.

2. In the ultrasonic diagnostic imaging system of Claim 1, further comprising a user operated control for manipulating said cursor into visual alignment with said displayed vessel or organ.

3. In the ultrasonic diagnostic imaging system of Claim 2, wherein said user operated control further comprises means for adjusting the size of said second component to align with said displayed vessel or organ.

4. In the ultrasonic diagnostic imaging system of Claim 3, wherein said user operated control further comprises means for adjusting the size of said second component to align with the diameter of said displayed vessel or organ.

5. In the ultrasonic diagnostic imaging system of Claim 3, wherein said cursor comprises third and fourth components at the ends of said second component; and wherein said user operated control further comprises means for aligning said third and fourth components with features of said displayed vessel or organ.

6. In the ultrasonic diagnostic imaging system of Claim 5, wherein said user operated control further comprises means for aligning said third and fourth components with walls of said displayed vessel or organ.

7. In the ultrasonic diagnostic imaging system of Claim 1, wherein said cursor further comprises means for delineating a Doppler sample volume in said displayed vessel or organ.

8. In the ultrasonic diagnostic imaging system of Claim 7, wherein said sample volume includes that portion of said displayed vessel or organ which is in alignment with said first and second components.

9. In an ultrasonic diagnostic imaging system which produces an ultrasonic display of blood vessels or organs, apparatus for measuring the volume flow of blood through a displayed blood vessel or organ comprising:
a user controlled cursor for delineating the direction of flow and dimension of a displayed blood vessel or organ;
an area processor, responsive to said radius or diameter delineated by said cursor, for computing the cross sectional area of said blood vessel or organ;
a Doppler processor, responsive to said delineated direction of flow, for computing the velocity of blood flow through said blood vessel or organ; and
a volume flow processor, responsive to said computed cross sectional area and velocity, for computing the volume flow of blood through said vessel or organ.

10. In the ultrasonic diagnostic imaging system of Claim 9, wherein said user controlled cursor comprises a first component for aligning with the direction of flow through said blood vessel or organ, and a second component, orthogonal to the first component, for aligning with the radius or diameter of said blood vessel or organ.

11. In the ultrasonic diagnostic imaging system of Claim 10, further comprising:
means for recording images of said displayed blood vessel or organ;
means for replaying said recorded images, whereby the alignment of said user controlled cursor with said direction of flow, blood vessel, or organ may be reviewed.

12. In the ultrasonic diagnostic imaging system of Claim 9, wherein said user controlled cursor further comprises means for delineating a Doppler sample volume in said blood vessel or organ.

13. In the ultrasonic diagnostic imaging system of Claim 9, further comprising a display for simultaneously displaying a two dimensional ultrasonic image of said blood vessel or organ, and a Doppler display of flow velocity through said blood vessel or organ.

14. In the ultrasonic diagnostic imaging system of Claim 9, wherein said volume flow processor computes a new volume flow calculation each cardiac cycle.

15. In the ultrasonic diagnostic imaging system of Claim 13, wherein said Doppler display comprises a spectral Doppler display which displays at least a complete cardiac cycle of velocity measurements; and wherein said volume flow processor computes a new volume flow calculation each cardiac cycle.

16. In an ultrasonic diagnostic imaging system which produces an ultrasonic display of blood vessels or organs, apparatus for measuring a dimension of a displayed blood vessel or organ comprising:
a user controlled cursor for delineating a dimension of a displayed blood vessel or organ; and
an M-mode display, responsive to the delineation of said dimension by said cursor, for displaying a time sequence of M-lines including said delineated dimension.

17. In the ultrasonic diagnostic imaging system of Claim 16, further comprises a tracking system, responsive to said delineation of a dimension of a displayed blood vessel or organ, for maintaining alignment of said cursor with said delineated dimension.

18. In the ultrasonic diagnostic imaging system of Claim 16, further comprising a two dimensional image display for displaying a blood vessel or organ; wherein said user controlled cursor delineates a dimension of a blood vessel or organ on said image display.

19. A method for measuring the volume of an organ of the body, comprising the steps of:
producing a two dimensional image of an organ;
positioning a cursor over said organ image, said cursor having an M-line in one direction and an adjustable length line in an orthogonal direction; and
using M-line data of said M-line and length data of said length line to compute a volume of said organ.

20. The method of Claim 19, wherein the step of using M-line data and length data comprises fitting said M-line data and said length data to dimensions of a predetermined shape.

21. The method of Claim 20, wehrein said predetermined shape comprises an ellipse.

22. In an ultrasonic diagnostic imaging system which produces an ultrasonic display of blood vessels or organs, apparatus for measuring the volume flow of blood through a displayed blood vessel or organ comprising:
a user controlled cursor for delineating a dimension of a displayed blood vessel or organ;
a tracking system, responsive to said delineation of a dimension of a displayed blood vessel or organ, for maintaining alignment of said cursor with said delineated dimension; and
a volume flow processor, responsive to said delineated dimension, for computing the volume flow of blood through said vessel or organ.

23. In the ultrasonic diagnostic imaging system of Claim 22, further comprising a Doppler processor for computing the velocity of blood flow through said blood vessel or organ; and wherein said volume flow processor is further responsive to said computed velocity for computing volume flow.

24. In the ultrasonic diagnostic imaging system of Claim 23, further comprising a display for producing a two dimensional display of said blood vessel or organ and a spectral display of flow velocities through said vessel or organ,
wherein said cursor is controlled by a user to delineate a dimension of said blood vessel or organ on said two dimensional display.

25. In the ultrasonic diagnostic imaging system of Claim 23, wherein said cursor further comprises means for delineating the direction of flow through said blood vessel or organ; wherein said Doppler processor is responsive to said delineated direction of flow; and wherein said delineated dimension is diameter of said vessel or organ.

26. In the ultrasonic diagnostic imaging system of Claim 23, wherein said volume flow processor computes a volume flow measurement using a delineated dimension of said blood vessel or organ and a substantially contemporaneous velocity computation.

27. In the ultrasonic diagnostic imaging system of Claim 26, wherein said volume flow processor produces an average volume flow measurement by averaging a plurality of said volume flow measurements over a cardiac cycle.

28. An ultrasonic diagnostic imaging system for measuring the volume flow of blood through a displayed blood vessel or organ comprising:
a display for simultaneously displaying
a two dimensional image of a blood vessel or organ;
a user controllable cursor for indicating a location in said blood vessel or organ image where a volume flow measurement is to be made and for delineating a dimension of said blood vessel or organ;
a Doppler display of flow velocities at said indicated location over at least a cardiac cycle; and
a volume flow measurement; and
a volume flow processor, responsive to said indicated location and dimension and said flow velocities for producing an updated volume flow measurement each cardiac cycle.

29. The ultrasonic diagnostic imaging system of Claim 28, wherein said delineated dimension is the diameter of said blood vessel or organ at said indicated location.

30. The ultrasonic diagnostic imaging system of Claim 28, wherein said display further comprises means for visually indicating the cardiac cycle of said Doppler display corresponding to the currently updated and displayed volume flow measurement.

31. In an ultrasonic diagnostic imaging system which produces an ultrasonic display of blood vessels or organs, apparatus for measuring a dimension of a displayed blood vessel or organ comprising:
means for delineating the diameter of a blood vessel or organ; and
means for automatically delineating a sample volume which encompasses said delineated diameter.

32. In the ultrasonic diagnostic imaging system of Claim 31, wherein said means for delineating the diameter comprises a first displayed cursor, and wherein said means for automatically delineating comprises a second displayed cursor.

33. In the ultrasonic diagnostic imaging system of Claim 31, wherein said means for automatically delineating comprises means for automatically delineating a sample volume in the plane of an ultrasonic image; and further comprising means for delineating a sample volume in the elevation dimension.

34. In the ultrasonic diagnostic imaging system of Claim 33, wherein said means for delineating a sample volume in the elevation dimension comprises means for adjusting the beamwidth of an ultrasound beam to encompass the diameter of a vessel in the elevation dimension.

35. In the ultrasonic diagnostic imaging system of Claim 33, further comprising means for making a Doppler measurement based upon the delineated dimensions of said sample volume in the image plane and elevation dimensions.

36. In the ultrasonic diagnostic imaging system of Claim 34, wherein said means for adjusting the beamwidth of an ultrasound beam comprises means for automatically adjusting the beamwidth of an ultrasound beam to encompass the diameter of a vessel in the elevation dimension.
